# EUROPÄISCHE PATENTSCHRIFT

(11) **EP 1 889 600 B1**
(45) Veröffentlichungstag und Bekanntmachung des Hinweises auf die Patenterteilung: **25.07.2012**
(21) Anmeldenummer: 06113321.1
(22) Anmeldetag: 28.04.2006
(51) Int. Cl.: A61K 8/73, A61K 8/34, A61Q 11/00, A61K 6/00, A61K 8/42

(54) **Verfahren zum Aufhellen von Zähnen**
Method for teeth whitening
Méthode pour le blanchiment des dents

(43) Veröffentlichungstag der Anmeldung: 20.02.2008
(73) Patentinhaber: Ivoclar Vivadent AG, 9494 Schaan (LI)
(72) Erfinder: Huwig, Alexander, 68729 Schwetzingen (DE); David, Gabriele, 88131, Lindau (DE); Marques, Duarte, 135-119, Lisboa (PT); Mata, Antonio, 1250-073, Lisboa (PT)
(74) Vertreter: UEXKÜLL & STOLBERG

(56) Entgegenhaltungen:
- EP-A- 0 511 782
- EP-A- 1 555 008
- WO-A-2005/070378

## Beschreibung

Die vorliegende Erfindung betrifft ein Verfahren zum Aufhellen der Zähne.

Zahlreiche Nahrungs- und Genußmittel, wie Tee, Kaffee, Rotwein und Tabak, können zu Verfärbungen der Zähne führen. Auch bestimmte Arzneimittel, wie zum Beispiel Tetrazyklin oder die Aufnahme von großen Mengen an Fluorid können Verfärbungen der Zähne hervorrufen. Da der Mund als Blickfang im Zentrum des Gesichts in einem hohen Maß das äußere Erscheinungsbild bestimmt, werden derartige Verfärbungen oft als eine Beeinträchtigung empfunden und Mittel und Verfahren zu deren Beseitigung finden zunehmendes Interesse.

Ein häufig angewendetes Verfahren umfaßt das Bleichen der Zähne mit konzentrierter Wasserstoffperoxidlösung (30 bis 37 %). Zur Förderung der oxidativen Wirkung des Peroxids werden Hitze und Licht angewendet. Mit diesem Verfahren lassen sich zwar Aufhellungen der Zähne erreichen, es erfordert jedoch einen hohen Aufwand, und die Verwendung einer relativ hohen Wirkstoffkonzentration kann Nebenwirkungen wie eine Irritation der Schleimhaut oder hypersensible Zähne verursachen.

Die US 4,032,627 offenbart pigmenthaltige Lacke zum Überdecken von Verfärbungen. Diese Lacke weisen eine geringe mechanische Belastbarkeit auf und müssen praktisch täglich neu aufgetragen werden.

Die US 5,290,566 offenbart gelförmige Mittel zum Aufhellen von Zähnen, die als aktiven Bestandteil 22 bis 32 Gew.-% Carbamidperoxid enthalten. Diese Gele werden auf eine Schiene aufgebracht, die dann auf die zu behandelnden Zähne aufgesetzt wird. Diese Schiene muß täglich mindestens 6 Stunden getragen werden, bis der gewünschte Aufhellungsgrad erzielt worden ist.

Aus der US 5,171,564 sind pastenförmige Zusammensetzungen bekannt, die neben Carbamidperoxid Schleifmittel enthalten.

Die US 5,425,953 offenbart flüssige Polymerzusammensetzungen, die neben einem Oxidationsmittel wie Carbamidperoxid ein wasserlösliches Cellulosepolymer und vorzugsweise auch einen Stabilisator wie Calciumdinatriumedetat enthalten. Die Zusammensetzungen werden ein- bis dreimal täglich auf die Zähne aufgebracht und bilden dort nach dem Verdampfen des Lösungsmittels einen Film, der das Oxidationsmittel freisetzt und sich innerhalb von etwa 1 Stunde zersetzt.

Weiterhin sind aus der US 6,083,421 lackförmige Bleichmittel auf der Basis von Carbamidperoxid bekannt, die für mehr als 1 Stunde an der Zahnoberfläche haften sollen. Bevorzugte Filmbildner sind Polyvinylbutyral, Cumaronharz und Schellack. Die Lacke weisen nach dem Trocknen nur eine geringe mechanische Stabilität auf und werden beim Berühren mit der Zunge oder der Mundschleimhaut leicht zerstört.

Schließlich beschreiben die DE 20 2004 000 552 U1 und die EP 1555 008 A1 orale Zusammensetzungen, die neben einem pharmazeutisch annehmbaren Oxidationsmittel und Lösungsmittel wasserunlösliche Alkylcellulose als Lackbildner enthält. Die Zusammensetzungen eignen sich insbesondere zur Anwendung durch den Patienten selbst, und es wird eine zweimal tägliche Anwendung über einen Zeitraum von vierzehn Tagen empfohlen.

Lackförmige Bleichmittel sind in der Regel gut zu handhaben, und ihre Anwendung ist für den Patienten mit relativ wenigen Unannehmlichkeiten verbunden. Nachteilig an den bekannten Verfahren zum Aufhellen von Zähnen ist jedoch die häufig nur durchschnittliche Bleichwirkung, so daß ein Bedarf an weiter verbesserten Verfahren zum Bleichen von Zähnen besteht.

Erfindungsgemäß wird diese Aufgabe durch ein Verfahren zum Aufhellen von Zähnen gelöst bei dem man
(a) ein Bleichmittel auf die Oberfläche des oder der zu bleichenden Zähne aufträgt,
(b) man das Bleichmittel für einen Zeitraum von 3 bis 20 Minuten auf den Zahn einwirken läßt,
(c) man das Bleichmittel von der Zahnoberfläche entfernt,
(d) man die Schritte (a) bis (c) mindestens einmal wiederholt, wobei das Verfahren 1 bis 3 Sitzungen umfaßt und man in jeder Sitzung die Schritte (a) bis (d) durchführt und man die Schritte (a) bis (c) bis zu neunmal pro Sitzung wiederholt.

Vorzugsweise läßt man das Bleichmittel in Schritt (b) für 5 bis 10 Minuten auf den Zahn einwirken.

Das erfindungsgemäße Verfahren kann in einer oder mehreren Sitzungen durchgeführt werden, wobei eine Sitzung dadurch gekennzeichnet ist, daß die Verfahrensschritte (a) bis (d) jeweils mindestens einmal durchlaufen werden.

Pro Sitzung werden die Schritte (a) bis (c) vorzugsweise - bis zu fünfmal und ganz besonders bevorzugt zweimal wiederholt.

Wenn mehr als eine Sitzung durchgeführt wird, beträgt der zeitliche Abstand zwischen zwei aufeinanderfolgenden Sitzungen vorzugsweise 4 bis 10 Tage, besonders bevorzugt 6 bis 8 Tage und ganz besonders bevorzugt eine Woche.

Die Dauer und Anzahl der Wiederholungen wird so bemessen, daß die gesamte Einwirkzeit des Bleichmittels pro Sitzung 45 bis 90 Minuten und ganz besonders bevorzugt etwa 60 Minuten beträgt. Umfang und Dauer der Behandlung richten sich nach der gewünschten Aufhellung der Zähne.

Es wurde überraschend gefunden, daß das erfindungsgemäße Verfahren in nur ein bis zwei Sitzungen eine Aufhellung der Zähne um bis zu 10 Farbschattierungen ermöglicht. Dies ist insofern erstaunlich, als diese Aufhellung auch mit bekannten Bleichmitteln erzielt wird, die bei der Verwendung bekannter Behandlungsschemas nur eine geringe Aufhellung ergeben. Das Aufhellen der Zähne dient rein kosmetischen Zwecken, es ist mit keiner parodontitis- oder kariesprophylaktische Wirkung verbunden.

Bevorzugt ist eine Aufhellungen von mindestens 6, vorzugsweise mindestens 8 und besonders bevorzugt mindestens 10 Farbschattierungen. Zur Bestimmung der Aufhellungen wird zunächst die Ausgangssituation dokumentiert, indem die aktuelle Zahnfarbe anhand einer Farbskala wie zum Beispiel dem Farbschlüssel VITAPAN^{®} classic bestimmt und fotografisch dokumentiert wird (Aufnahme mit Referenzprobe im Bild). Nach dem erfindungsgemäßen Verfahren wird erneut die aktuelle Situation ermittelt und dokumentiert.

Zur Gewährleistung einer gleichmäßigen, fleckenfreien Aufhellung des oder der behandelten Zähne wird das erfindungsgemäße Verfahren vorzugsweise auf gereinigte Zähne angewendet, insbesondere auf Zähne die frei von Zahnbelag sind, d.h. Zähnen, die von harten und weichen Belägen und Plaqueresten befreit wurden. Hierzu können die Zähne vor der Durchführung des erfindungsgemäßen Verfahrens einer professionellen Zahnreinigung durch den Zahnarzt oder eine geschulte Zahnarzthelferin unterzogen werden.

Um eine Reizung von Weichgewebe wie Gingiva und Mundschleimhaut durch das verwendete Bleichmittel zu vermeiden oder um Zähne, die nicht gebleicht werden sollen, zu schützen, können Bereiche, die nicht mit dem Bleichmittel in Kontakt kommen sollen, insbesondere das Zahnfleisch, der Zahnfleischrand und sensible Zähne mit einem Abdeckmittel abdeckt werden. Das Abdeckmittel muß zur Anwendung im Mund des Patienten geeignet sein. Ein bevorzugtes Abdeckmittel ist Vaseline, es kommen aber auch andere dentale Einfett- und Isoliermittel in Betracht. Außerdem ist der Einsatz eines Kofferdams zum Schutz von Zahnfleisch und Mundschleimhaut bevorzugt. Durch den Einsatz eines Abdeckmittels wird ein Eindringen des Bleichmittels in die parodontalen Taschen verhindert.

Gemäß einer bevorzugten Ausführungsform des erfindungsgemäßen Verfahrens wird bei seiner Durchführung ein Lippen- und Wangenhalter eingesetzt, wodurch die an die Zahnreihen zum Anliegen kommenden Lippen und Wangen von den Zahnreihen abgehalten werden und ein ausreichender Arbeitsraum freigemacht wird. Vorzugsweise wird ein Lippen- und Wangenexpander verwendet, der zwei Spannrahmen sowie die Spannrahmen verbindende, nicht profilierte folienartige Mittel aufweist. Derartige Lippen- und Wangenhalter werden beispielsweise in der WO 03/051185 beschrieben und sind bei der Ivoclar Vivadent AG unter der Bezeichnung OptraGate^{®} erhältlich.

Es wurde gefunden, daß die Verwendung des Bleichmittels in Kombination mit einem Lippen- und Wangenhalter zu überraschend hohen Aufhellungen der Zähne führt, wenn der Lippen- und Wangenhalter nicht nur beim Auftragen des Bleichmittels eingesetzt ist, sondern während der gesamten Einwirkzeit des Bleichmittels im Mund gelassen wird. Selbst bei Verwendung von Bleichlacken mit geringen Oxidationsmittelgehalten konnten hohen Aufhellungen erzielt werden. Außerdem erleichtert das durch den Lippen- und Wangenhalter geschaffene freie Arbeitsfeld die gezielte Aufhellung einzelner Zähne und das Entfernen des Bleichmittels.

Weiterhin ist es bevorzugt, die zu bleichende Zahnoberfläche vor dem Auftragen des Bleichmittels zu trocknen. Das Trocknen kann z.B. durch Anblasen mit einem Luftstrahl oder durch Abtupfen mit einem Zellstofftuch erfolgen.

Das Auftragen des Bleichmittels in Schritt (a) auf die zu bleichende Zahnoberfläche kann mit einem Applikator, z.B. einem kleinen Pinsel, erfolgen. Im Hinblick auf eine mögliche Präzipitation des Lackbildners der oralen Zusammensetzung bei Speichelkontakt wird die benötigte Menge an Bleichmittel vorzugsweise von dem Verpackungsgefäß in beispielsweise eine kleine Schale überführt und dann mit dem Applikator auf die Zähne aufgebracht. Auf diese Weise wird ein Kontakt der in dem Verpackungsgefäß befindlichen Zusammensetzung mit dem benutzten Applikator vermieden.

Nach dem Auftragen läßt man das Bleichmittel in Schritt (b) auf den oder die Zähne einwirken. Hierzu läßt man das Bleichmittel vorzugsweise trocknen. Das Trocknen kann durch das Anblasen mit einem Luftstrahl beschleunigt werden. Im Fall de bevorzugten Bleichmittel bildet sich beim Verdampfen des Lösungsmittels eine farblose bis weiße oder ggf. gefärbte Lackschicht, die fest an der Zahnoberfläche haftet und für die gewünschte Zeit im Mund verbleibt.

Nach Ablauf der Einwirkzeit wird der gebildete Lack in Schritt (c) entfernt. Die erfindungsgemäß bevorzugten Bleichmittel bilden eine Lackschicht, die wie ein Film abgezogen oder mit einer nassen Zahnbürste entfernt werden können. Es ist jedoch auch möglich, zum Entfernen der Lackschicht ein Ultraschall- oder ein langsam rotierendes, nicht schneidendes Gerät zu verwenden.

Erfindungsgemäß werden die Schritte (a) bis (c) einmal oder mehrfach wiederholt.

Erfindungsgemäß werden Bleichmittel verwendet, die die folgenden Komponenten enthalten:
(i) 50 - 96 Gew.-% Lösungsmittel,
(ii) 2 - 30 Gew.-% pharmazeutisch annehmbares Oxidationsmittel und
(iii) 2 - 30 Gew.-% Lackbildner.

Es handelt sich bei diesen Bleichmitteln um Lacke. Sie werden im Folgenden daher auch als Bleichlacke bezeichnet.

Bevorzugte pharmazeutisch annehmbare Oxidationsmittel sind Natriumperborat, Chlordioxid und pharmazeutisch annehmbare Peroxidverbindungen, wie z.B. organische Peroxide, vorzugsweise Methylperoxid, Ethylperoxid und insbesondere Glycerinperoxid oder Benzylperoxid. Besonders bevorzugte Peroxide sind Wasserstoffperoxid und Carbamidperoxid sowie Mischungen dieser beiden Substanzen. Carbamidperoxid wird vorzugsweise in einer Menge von 4 bis 20 Gew.-%, besonders bevorzugt 5 bis 10 Gew.-% und ganz besonders bevorzugte in einer Menge von 5,3 bis 6,5 Gew.-% verwendet. Wasserstoffperoxid wird vorzugsweise in einer Menge von 2 bis 20 Gew.-%, besonders bevorzugt 5,5 bis 13 Gew.-% und am meisten bevorzugt 5,5 bis 12 Gew.-% eingesetzt, gemessen als H₂O₂.

Wasserstoffperoxid wird vorzugsweise in Form einer kommerziell erhältlichen Wasserstoffperoxid-Lösung in Wasser mit einer H₂O₂-Konzentration von 34,5 bis 36,5% eingesetzt. Für höhere Wasserstoffperoxidkonzentrationen wird vorzugsweise eine ebenfalls kommerziell erhältliche wäßrige H₂O₂-Lösung mit einer Konzentration von 48 bis 70% verwendet.

Carbamidperoxid hydrolysiert in Gegenwart von Wasser zu Harnstoff und Wasserstoffperoxid. Der gebildete Harnstoff hat den Vorteil, daß er pH-neutralisierend wirkt. Das freigesetzte Wasserstoffperoxid diffundiert in den Zahnschmelz und das Dentin, wo es bei der Oxidation zu Wasser und Sauerstoff zerfällt. Durch die Diffusion des Wasserstoffperoxids in den Zahnschmelz wird das chemische Gleichgewicht zwischen Carbamidperoxid und seinen Zerfallsprodukten gestört, was die weitere Freisetzung von Wasserstoffperoxid aus dem Carbamidperoxid zur Folge hat. Durch die erfindungsgemäßen Lacke wird sichergestellt, daß das Wasserstoffperoxid praktisch vollständig in den Zahnschmelz diffundiert und nicht wie bei herkömmlichen Lacken zu großen Teilen in Speichel und Weichgewebe abgegeben wird.

Um den pH-Wert-neutralisierenden Effekt des im Carbamidperoxid enthaltenen Harnstoffs auszunutzen können auch Mischungen von Wasserstoffperoxid und Carbamidperoxid eingesetzt werden. Beide Peroxid-Verbindungen werden hierzu vorzugsweise in einer Menge von bis zu jeweils 20 Gew-% eingesetzt. Besonders bevorzugt wird Wasserstoffperoxid in einer Menge von 4 bis 20 Gew.-% und Carbamidperoxid in einer Menge von 5 bis 10 Gew.-% eingesetzt.

Bei den erfindungsgemäß verwendeten Zusammensetzungen handelt es sich um Lacke, die vorzugsweise eine Viskosität von 5 bis 20.000 mPas, besonders bevorzugt von 100 bis 5.000 mPas und ganz besonders bevorzugt von 1.000 bis 5.000 mPas aufweisen. Falls nicht anders angegeben, handelt es sich bei allen hierin angegebenen Viskositäten um Werte, die mit einem Rotationsviskosimeter bestimmt wurden (gemessen bei 23,0°C, Scherrate 100s⁻¹, Meßsystem Kegel-Platte, Kegeldurchmesser 20 mm, Öffnungswinkel 2,5°, Spalt 70 µm).

Die Einstellung der Viskosität erfolgt durch die Zugabe des Lackbildners. Bevorzugte Lackbildner sind Lackbildner auf Cellulosebasis, insbesondere Alkylcellulose.

Vorzugsweise wird eine wasserunlösliche Alkylcellulose verwendet, d.h. Alkylcellulose mit einer Löslichkeit von maximal 0,1 g Alkylcellulose pro 100 g Wasser bei Raumtemperatur. Besonders bevorzugt ist Ethylcellulose, insbesondere Ethylcellulose mit einem mittleren Substitutionsgrad (DS) von > 1,5, besonders bevorzugt > 2,0, insbesondere 2,0 bis 2,6, und ganz besonders bevorzugt 2,3 bis 2,4. In Cellulose weist jede Glucoseeinheit drei freie Hydroxylgruppen auf. Sind alle drei Hydroxylgruppen aller Glucoseeinheiten der Cellulose durch Alkoxygruppen substituiert, beträgt der Substitutionsgrad (DS) 3. Ist nur ein Teil der Hydroxylgruppen der Glucoseeinheiten mit Alkoxygruppen substituiert, ist der Substitutionsgrad entsprechend geringer. Der Substitutionsgrad DS gibt die durchschnittliche Anzahl an substituierten Hydroxylgruppen pro verknüpfter Glucoseeinheit an. Ganz besonders bevorzugt ist Ethylcellulose, die einen Ethoxylgehalt von 45 bis 50 % aufweist.

Die erforderliche Menge an Lackbildner hängt von der Art des Lackbildners ab und wird vorzugsweise so bemessen, daß der Lack eine Viskosität innerhalb der oben angegebenen Bereiche hat. Im Fall der vorzugsweise als Lackbildner verwendeten Alkylcellulose hängt die Menge unter anderem von deren Kettenlänge und Molekulargewicht und somit von der in Lösung gebildeten Viskosität ab. Alkylcellulose wird vorzugsweise in einer Menge von 2 bis 30 Gew.-%, besonders bevorzugt 5 bis 20 Gew.-%, ganz besonders bevorzugt 6 bis 12 Gew.-% eingesetzt. Die Ermittlung der erforderlichen Mengen an anderen Lackbildnern zur Einstellung der Viskosität der Bleichlacke ist dem Fachmann aufgrund seiner Fachkenntnisse ohne weiteres möglich.

Bevorzugte Lösungsmittel sind Diethylehter, Aceton, Ester, wie beispielsweise Ethylacetat, und Alkohole, wie Isopropanol und insbesondere Ethanol. Besonders bevorzugte Lösungsmittel sind Ethylacetat und Ethanol. Mischungen aus einem oder mehreren dieser Lösungsmittel können verwendet werden. Darüber hinaus kann das Lösungsmittel bis zu 30 Gew.-% an Wasser enthalten, bezogen auf die Gesamtlösungsmittelmenge, d.h. auf die Menge an Wasser und weiterem Lösungsmittel. Bei der Verwendung von Carbamidperoxid liegt der Wassergehalt vorzugsweise unter 12 Gew.-%, besonders bevorzugt unter 2 Gew.-%, wobei wasserfreie Zusammensetzungen am meisten bevorzugt sind.

Gemäß einer Ausführungsform enthalten die Zusammensetzungen neben den genannten Komponenten zusätzlich eine pharmazeutisch annehmbare Säure, vorzugsweise Phosphorsäure (H₃P0₄), Pyrophosphorsäure (H₄P₂0₇) und insbesondere Zitronensäure. Die Säure wird vorzugsweise in einer Menge von 0,05 bis 5,0 Gew.-%, insbesondere 0,5 bis 5 Gew.-%, besonders bevorzugt 0,3 bis 0,6 Gew.-% eingesetzt. Alle Prozentangaben beziehen sich, wenn nicht anders angegeben, auf die Gesamtmasse der Zusammensetzung. Durch die Säure wird eine feste und gleichmäßige Haftung des Lacks an der Zahnoberfläche sichergestellt, ohne jedoch den Zahn zu schädigen.

Als weitere optionale Komponente enthalten die erfindungsgemäß verwendeten Zusammensetzungen vorzugsweise auch Dexpanthenol, vorzugsweise in einer Menge von 0,1 bis 1,0 Gew.-%, besonders bevorzugt 0,2 bis 0,5 Gew.-%. Dexpanthenol ist ein Provitamin, das Irritationen und Reizungen der Mundschleimhaut entgegenwirkt, die durch die Peroxidverbindung, das Lösungsmittel oder die Säure ausgelöst werden können. Zusammensetzungen mit einem Gehalt an Dexpanthenol sind daher in der Regel besser verträglich als herkömmliche Mittel.

Darüber hinaus können der Zusammensetzung weitere Komponenten, wie Aromastoffe, Geschmacksstoffe und/oder Süßstoffe zugesetzt werden. Diese Komponenten werden vorzugsweise in einer Menge von jeweils 0,01 bis 0,5 Gew.-%, besonders bevorzugt 0,02 bis 0,2 Gew.-% und ganz besonders bevorzugt 0,03 bis 0,06 Gew.% verwendet.

Bevorzugte Aroma- und Geschmacksstoffe sind Methylsalicylat, Fenchelholzöl, Nelkenöl, Salbeiöl, Eukalyptusöl, Majoranöl, Menthol und vorzugsweise Minzöl, d.h. Pfefferminz- und/oder Krauseminzöl. Besonders bevorzugt sind synthetische Minzöle und -aromen, insbesondere die von der Firma Symrise GmbH unter der Bezeichnung OPTAMINT^{®} vertriebenen Öle und Aromen. Weitere geeignete Aromen sind Erdbeer-, Himbeer-, Bananen-, Kirsch-, Caramel-, Birnen-, Apfel-, Zitronen- und Pfirsicharoma sowie Orangenöl und Grapefruitaroma.

Als Süßstoffe eignen sich besonders Natriumsaccharin, Natriumzyklamat, Xylitol, Aspartam und dergleichen. Natriumsaccharin ist besonders bevorzugt.

Bei den erfindungsgemäß verwendeten Zusammensetzungen handelt es sich um farblose bis weiße Lacke, die gemäß einer bevorzugten Ausführungsform neutral sind, d.h. einen pH-Wert im Bereich von 5 bis 8, vorzugsweise 6, 5 bis 7, 5 aufweisen, gemessen an einer wäßrigen Dispersion der Zusammensetzung (1 bis 25 Gew.-%). Die Lacke tropfen nicht, ziehen beim Auftragen keine Fäden und lassen sich mit einem Pinsel präzise auftrage. Sie trocknen innerhalb von etwa 1 Minute.

Säurehaltige Zusammensetzungen weisen vorzugsweise einen pH-Wert von 1 bis 6, besonders bevorzugt 2 bis 4 und ganz besonders bevorzugt 2 bis 3 auf.

Die Zusammensetzungen können gemäß einer Ausführungsform auch Farbstoffe enthalten, die keinen negativen Einfluß auf die Bleichwirkung haben, beispielsweise Carmin und Carthamin. Durch das Verwenden gefärbter Zusammensetzungen läßt sich leichter sicherstellen, daß die Zahnoberfläche vollständig benetzt ist. Die Zusammensetzungen enthalten vorzugsweise keine Schleifmittel.

Bevorzugte Zusammensetzungen enthalten die folgenden Komponenten:
(i) 50,0 bis 96,0 Gew.-%, vorzugsweise 60 bis 90 Gew.-%, insbesondere 80 bis 88 Gew.-%, ganz besonders bevorzugt 84 bis 86 Gew.-% Lösungsmittel, vorzugsweise Ethanol,
(ii) 2 bis 20 Gew.-% Oxidationsmittel, insbesondere 5,3 bis 6,5 Gew.-% Carbamidperoxid oder 6 bis 20 Gew.-% Wasserstoffperoxid,
(iii)2 bis 30 Gew.-%, vorzugsweise 8,0 bis 15,0 Gew.-%, insbesondere 9 bis 12 Gew.-% Lackbildner, vorzugsweise Alkylcellulose, besonders bevorzugt Ethylcellulose,
(iv) 0 bis 5,0 Gew.-%, insbesondere 0,5 bis 5 Gew.-%, besonders bevorzugt 0,3 bis 0,6 Gew.-% Säure, vorzugsweise Zitronensäure,
(v) 0 bis 1,0 Gew.-%, insbesondere 0,2 bis 0,5 Gew.-% Dexpanthenol,
(vi) 0 bis 0,5 Gew.-%, insbesondere 0 bis 0,2 Gew.-%, besonders bevorzugt 0,01 bis 0,05 Gew.-% Geschmacks- und/oder Aromastoff, insbesondere Minzöl oder Minzaroma.

Ein besonders bevorzugtes Bleichmittel auf der Basis von Wasserstoffperoxid weist folgende Zusammensetzung auf:
(i) 55 bis 80 Gew.-%, insbesondere 62 bis 80 Gew.-% Ethanol,
(ii) 5,5 bis 20,0 Gew.-%, insbesondere 6 bis 20 Gew.-% Wasserstoffperoxid,
(iii) 5,5 bis 12,5 Gew.-%, insbesondere 8,5 bis 9,5 Gew.-% Ethylcellulose,
(iv) 0 bis 28 Gew.-%, insbesondere 6 bis 12 Gew.-% Wasser,
(v) 0,2 bis 0,5 Gew.-% Dexpanthenol und
(vi) 0,01 bis 0,06 Gew.-% Aroma.

Der Wassergehalt bezieht sich hier auf die Gesamtmasse der Zusammensetzung, wobei jedoch zusätzlich auch der oben definierte maximale Wassergehalt bezogen auf die Gesamtlösungsmittelmenge zu beachten ist.

Ein ganz besonders bevorzugtes Bleichmittel auf der Basis von Carbamidperoxid weist folgende Zusammensetzung auf:
(i) 79 bis 85 Gew.-% Ethanol,
(ii) 5,3 bis 6,5 Gew.-% Carbamidperoxid,
(iii)8,5 bis 9,5 Gew.-% Ethylcellulose,
(iv) 0 oder 0,3 bis 0,6 Gew.-% Zitronensäure,
(v) 0,2 bis 0,5 Gew.-% Dexpanthenol und
(vi) 0,01 bis 0,06 Gew.-% Aroma.

Die erfindungsgemäß verwendeten Zusammensetzungen werden hergestellt, indem die Komponenten in den gewünschten Mengen miteinander gemischt werden, bis eine homogene, streichfähige Lösung mit der gewünschten Viskosität erhalten wird.

Gegenstand der Erfindung ist auch die Verwendung eines pharmazeutisch annehmbaren Oxidationsmittels zur Herstellung eines Mittels zum Aufhellen von Zähnen gemäß dem Behandlungsschema, bei dem man:
(a) ein Bleichmittel auf die Oberfläche des oder der zu bleichenden Zähne aufträgt,
(b) man das Bleichmittel für einen Zeitraum von 3 bis 30 Minuten auf den Zahn einwirken läßt,
(c) man das Bleichmittel von der Zahnoberfläche entfernt,
(d) man die Schritte (a) bis (c) mindestens einmal wiederholt.

Bevorzugte Ausgestaltungen des Behandlungsschemas ergeben sich aus der obigen Beschreibung des erfindungsgemäßen Verfahrens.

Zur Durchführung des Verfahrens eignet sich z.B. ein Kit zum Aufhellen von Zähnen, der die zur Durchführung des erfindungsgemäßen Verfahrens erforderlichen Materialien enthält, insbesondere
(A) ein Bleichmittel,
(B) einen Lippen- und Wangenhalter.

Vorzugsweise enthält der Kit zusätzlich auch (C) einen Applikator zum Auftragen des Bleichmittels auf den oder die Zähne und/oder (D) ein Abdeckmittel.

Bevorzugte Ausführungsformen des erfindungsgemäßen Kits sind solche, in denen die Komponenten (A) bis (D) durch die oben beschriebenen, vorteilhaften Merkmale gekennzeichnet sind.

Als Applikator enthält der Kit vorzugsweise einen oder mehrere Pinsel. Weiterhin kann in der Verpackungseinheit auch (E) ein Gefäß, vorzugsweise eine kleine Schale, zur Aufnahme der für eine Auftragung erforderlichen Menge des Bleichmittels enthalten. Auch enthält der Kit vorzugsweise (F) eine Gebrauchsanleitung, die die Durchführung des erfindungsgemäßen Verfahrens beschreibt.

Im Folgenden wird die Erfindung anhand von Ausführungsbeispielen näher erläutert.

### Beispiele

### Beispiele 1-6: Herstellung von oralen Zusammensetzungen

Es wurden Bleichlacke mit den in Tabelle 1 angegebenen Zusammensetzungen hergestellt. Hierzu wurde Ethanol in ein Becherglas gegeben und mit Ethylcellulose versetzt. Die Mischung wurde solange gerührt, bis eine homogene Lösung vorlag. Danach wurden nacheinander Carbamidperoxid oder Wasserstoffperoxid, Dexpanthenol und Aroma zugesetzt und die Mischung gleichmäßig verrührt. Es wurden farblose bis weiße Lacke erhalten.

Gemäß den in den Beispielen 1 und 8 der beschriebenen Verfahren wurden die folgenden Bleichmittel hergestellt.

**Tabelle 1: Zusammensetzung in Gew.-% der hergestellten oralen Zusammensetzungen**

| **Komponente** | **Beispiel** | | | | | |
|---|---|---|---|---|---|---|
| | **1^{a}** | **2^{b}** | **3** | **4** | **5** | **6** |
| Ethanol^{c} | 84,7 | 70,6 | 78,67 | 72,67 | 66,67 | 62,10 |
| Oxidationsmittel | 5,9 (CP) | 6,0 (HP) | 6,00 (HP) ^{d} | 9,00 (HP) ^{d} | 12,00 (HP) ^{d} | 20,00 (HP) ^{e} |
| Wasser | - | 14,0 | 6,00 | 9,00 | 12,00 | 8,57 |
| Ethylcellulose^{f} | 9,0 | 9,0 | 9,00 | 9,00 | 9,00 | 9,00 |
| Dexpanthenol | 0,3 | 0,3 | 0,30 | 0,30 | 0,30 | 0,30 |
| Aroma^{g} | 0,1 | 0,1 | 0,03 | 0,03 | 0,03 | 0,03 |

| | | | | | | |
|---|---|---|---|---|---|---|
| (CP): enthält Carbamidperoxid als Oxidationsmittel (HP): enthält Wasserstoffperoxid als Oxidationsmittel ^{a}_{:} gemäß DE 20 2004 000 552 U1 ^{b}: gemäß DE 20 2004 000 552 U1 ^{c}: Ethanol puriss p.a. ^{d} . Verwendung einer 50%igen Wasserstoffperoxidlösung zur Herstellung ^{e}_{:} Verwendung einer 70%igen Wasserstoffperoxidlösung zur Herstellung ^{f}: Ethylcellulose EC 100 ^{g}: Optamint^{®} Krauseminze | | | | | | |

### Beispiel 7: Bleichen von Zähnen

Die Untersuchungen wurden an insgesamt ca. 380 freiwilligen Probanden durchgeführt.

Von allen Probanden wurde eine allgemeine Anamnese aufgenommen und ein zahnärztlicher Befund erstellt. Bei allen Probanden wurde vor Behandlungsbeginn eine professionelle Zahnreinigung durchgeführt. Erst nach der Zahnreinigung wurde die Ausgangssituation dokumentiert und die aktuelle Zahnfarbe anhand des Vita Farbskala (VITAPAN^{®} classic) bestimmt und fotografisch dokumentiert (frontale Übersichtsaufnahme mit Vita-Referenzprobe im Bild).

Danach wurden die Probanden in Gruppen mit jeweils mindestens 10 Teilnehmern aufgeteilt.

Bei einer ersten Personengruppe (Gruppe 1) wurde die Behandlung mit dem oxidationsmittelhaltigen Bleichmittel aus Beispiel 3 auf die erfindungsgemäße Weise durchgeführt. Vor jeder Lackapplikation wurden das Zahnfleisch und hypersensible Zähne mit Vaseline abgedeckt. Die Oberflächen der zu bleichenden Zähne wurden dann mit einem Luftpuster getrocknet. Zur Erleichterung der Erreichbarkeit der zu behandelnden Zähne wurde ein Lippen-Wangenhalter gemäß WO 03/051185 (OptraGate^{®}, Fa. Ivoclar Vivadent) eingesetzt.

Der Lack wurde mit einem Pinsel in einer gleichmäßigen Schicht auf die Zahnoberflächen aufgetragen. Nach der Applikation wurde der Lippen-Wangen-Halter für die gesamte Einwirkzeit des Lacks im Munde des Patienten belassen. Der Patient wurde aufgefordert, für die Dauer der Einwirkzeit die Zähne nicht mit der Zunge zu berühren. Nach Ablauf der Einwirkzeit wurde der Lack mit einem Nylonbürstchen und Wasserspray entfernt und die Schleimhaut auf Irritationen hin untersucht. Erst danach wurde der Lippen-Wangen-Halter entfernt.

Die Personen wurden in zwei Sitzungen jeweils 6 Bleichmittelapplikationen mit einer Einwirkzeit von je 10 Minuten unterzogen. Die Gesamteinwirkzeit des Bleichlacks betrug damit 60 Minuten pro Sitzung. Die Sitzungen wurden im Abstand von einer Woche durchgeführt.

Bei jeder Sitzung wurde die aktuelle Situation ermittelt. Hierzu wurde die aktuelle Zahnfarbe entsprechend der Vita-Farbskala bestimmt und fotografisch dokumentiert. Bereits nach der ersten Sitzung wurde im Durchschnitt eine Aufhellung um 6,6 Farbschattierungen beobachtet (Standardabweichung 2,01, N=20). In der zweiten Sitzung wurde eine weitere Aufhellung um durchschnittlich 3,06 Farbschattierungen erreicht (Standardabweichung 1,8, N=16). Nach nur zwei Sitzungen wurde damit im Durchschnitt eine Aufhellung von knapp 10 Farbschattierungen gemäß der Vita-Farbskala erzielt.

Die zweite Gruppe (Gruppe 2) wurde mit einem Lack behandelt, der kein Oxidationsmittel enthielt, in seiner äußeren Erscheinungsform aber mit den oxidationsmittelhaltigen Zusammensetzungen identisch war (Placebo). Die Behandlung erfolge wie bei Gruppe (1) beschrieben. Bei dieser Gruppe wurde nach der ersten Sitzung eine Abnahme der Helligkeit um 0,4 4 Farbschattierungen gemäß Vita-Farbskala gefunden, nach der zweiten Sitzung eine Aufhellung um 0,8 Schattierungen, d.h. eine Aufhellung von 0,4 Farbschattierungen im Vergleich mit dem Ausgangszustand. Diese Werte sind nicht signifikant.

Demgegenüber ergab die Behandlung mit der oxidationsmittelhaltigen Zusammensetzung aus Beispiel 1 nach einem üblichen Behandlungsplans nur eine Aufhellung um durchschnittlich 1,6 Helligkeitsstufen (DE 20 2004 000 552 U1, Beispiel 7). Die Behandlung wurde über einen Zeitraum von zwei Wochen durchgeführt und umfaßte insgesamt 14 Bleichmittelapplikationen von jeweils 15 Minuten (Gesamteinwirkzeit: 210 Minuten). Die Gesamteinwirkzeit war damit etwa doppelt so hoch wie bei Gruppe 1, dennoch fiel die erzielte Aufhellung deutlich geringer aus.

## Patentansprüche

1. Verfahren zum Aufhellen von Zähnen, bei dem man
(a) ein Bleichmittel auf die Oberfläche des oder der zu bleichenden Zähne aufträgt, das die folgenden Bestandteile enthält:
(i) 50 - 96 Gew.-% Lösungsmittel,
(ii) 2 - 30 Ges.-% pharmazeutisch annehmbares Oxidationsmittel und
(iii) 2 - 30 Gew.-% Lackbildner, jeweils bezogen auf die Gesamtmasse der Zusammensetzung,
(b) man das Bleichmittel für einen Zeitraum von 3 bis 20 Minuten auf den Zahn einwirken läßt,
(c) man das Bleichmittel von der Zahnoberfläche entfernt,
(d) man die Schritte (a) bis (c) mindestens einmal wiederholt,
wobei das Verfahren 1 bis 3 Sitzungen umfaßt und man in jeder Sitzung die Schritte (a) bis (d) durchführt und man die Schritte (a) bis (c) bis zu neunmal pro Sitzung wiederholt und wobei man die Dauer und Anzahl der Wiederholungen so bemisst, dass die gesamte Einwirkzeit des Bleichmittels 45 bis 90 Minuten pro Sitzung beträgt.

2. Verfahren nach Anspruch 1, bei dem man das Bleichmittel in Schritt (b) für 5 bis 10 Minuten auf den Zahn/die Zähne einwirken läßt.

3. Verfahren nach Anspruch 1 oder 2, bei dem man die Schritte (a) bis (c) bis zu fünfmal, vorzugsweise zweimal pro Sitzung wiederholt.

4. Verfahren nach einem der Ansprüche 1 bis 3, das mindestens zwei Sitzungen umfaßt, wobei der zeitliche Abstand zwischen zwei aufeinanderfolgenden Sitzungen 4 bis 10 Tage, vorzugsweise 6 bis 8 Tage beträgt.

5. Verfahren nach einem der vorhergehenden Ansprüche, bei dem man die Dauer und Anzahl der Wiederholungen so bemißt, daß die gesamte Einwirkzeit des Bleichmittels 60 Minuten pro Sitzung beträgt.

6. Verfahren nach einem der vorhergehenden Ansprüche, bei dem die zu bleichenden Zähne frei von Zahnbelag sind.

7. Verfahren nach einem der vorhergehenden Ansprüche, bei dem man Bereiche, die nicht mit dem Bleichmittel in Kontakt kommen sollen, mit einem Abdeckmittel abdeckt, insbesondere das Zahnfleisch, den Zahnfleischrand und/oder sensible Zähne.

8. Verfahren nach Anspruch 7, bei dem man als Abdeckmittel Vaseline und/oder einen Kofferdam einsetzt.

9. Verfahren nach einem der vorhergehenden Ansprüche, bei dem man einen Lippen- und Wangenhalter einsetzt.

10. Verfahren nach einem der vorhergehenden Ansprüche, bei dem man die Zahnoberfläche vor dem Auftragen des Bleichmittels trocknet.

11. Verfahren nach einem der vorhergehenden Ansprüche, bei dem die Zusammensetzung als Lösungsmittel Alkohol, insbesondere Ethanol und/oder Isopropanol, Diethylether, Ester, insbesondere Ethylacetat, und/oder Aceton enthält, als pharmazeutisch annehmbares Oxidationsmittel eine Peroxidverbindung enthält, und als Lackbildner Alkylcellulose enthält.

12. Verfahren nach einem der Ansprüche 1 bis 11, bei dem die Zusammensetzung eine Viskosität von 5 bis 20.000 mPas aufweist.

13. Verfahren nach Anspruch 11, bei dem die Zusammensetzung eine Alkylcellulose mit einer Wasserlöslichkeit von maximal 0,1 g in 100 g Wasser bei Raumtemperatur enthält.

14. Verfahren nach einem der Ansprüche 1 bis 13, bei dem die Zusammensetzung zusätzlich Dexpanthenol, vorzugsweise in einer Menge von 0,1 bis 1,0 Gew.-%, bezogen auf die Gesamtmasse der Zusammensetzung, enthält.

## Claims

1. Method for brightening teeth, in which method
(a) a bleaching agent comprising the following components is applied to the surface of the tooth or teeth to be bleached:
(i) 50 - 96 wt % solvent,
(ii) 2 - 30 wt % of a pharmaceutically acceptable oxidising agent and
(iii) 2 - 30 wt % of a varnish former, each based on the total mass of the composition,
(b) the bleaching agent is left on the tooth for a period of 3 to 20 minutes,
(c) the bleaching agent is removed from the tooth surface,
(d) steps (a) to (c) are repeated at least once,
wherein the method comprises 1 to 3 sessions and steps (a) to (d) are carried out in each session and steps (a) to (c) are repeated up to nine times per session and wherein the duration and number of repetitions are such that the total contact time of the bleaching agent is 45 to 90 minutes per session.

2. Method according to claim 1, in which in step (b) the bleaching agent is left in contact with the tooth/teeth for 5 to 10 minutes.

3. Method according to claim 1 or 2, in which steps (a) to (c) are repeated up to five times, preferably twice per session.

4. Method according to one of claims 1 to 3, comprising at least two sessions, wherein the time interval between two consecutive sessions is 4 to 10 days, preferably 6 to 8 days.

5. Method according to one of the previous claims, in which the duration and number of repetitions are such that the total contact time of the bleaching agent is 60 minutes per session.

6. Method according to one of the previous claims, in which the teeth to be bleached are free of plaque.

7. Method according to one of the previous claims, in which areas that are not to come into contact with the bleaching agent, especially the gums, the gingival line and/or sensitive teeth, are covered with a masking material.

8. Method according to claim 7, in which Vaseline and/or a rubber dam is used as the masking material.

9. Method according to one of the previous claims, in which a lip and cheek retractor is used.

10. Method according to one of the previous claims, in which the tooth surface is dried before the application of the bleaching agent.

11. Method according to one of the previous claims, in which the composition comprises alcohol, in particular ethanol and/or isopropanol, diethyl ether, esters, in particular ethyl acetate, and/or acetone as the solvent, a peroxide compound as the pharmaceutically acceptable oxidising agent, and alkyl cellulose as the varnish former.

12. Method according to one of claims 1 to 11, in which the viscosity of the composition is in the range 5 to 20 000 mPas.

13. Method according to claim 11, in which the composition comprises an alkyl cellulose with a water solubility of at most 0.1 g in 100 g water at room temperature.

14. Method according to one of claims 1 to 13, in which the composition also comprises Dexpanthenol, preferably in a quantity of 0.1 to 1.0 wt %, relative to the overall mass of the composition.

## Revendications

1. Procédé de blanchiment des dents, dans lequel :
a) on dépose, sur la surface de la dent ou des dents à blanchir, un agent de blanchiment qui contient les constituants suivants :
i) de 50 à 96 % en poids d'un solvant,
ii) de 2 à 30 % en poids d'un agent oxydant pharmacologiquement admissible,
iii) et de 2 à 30 % en poids d'un agent formant un vernis,
dans chaque cas par rapport au poids total de la composition ;
b) on laisse l'agent de blanchiment agir sur la dent ou les dents, pendant un laps de temps de 3 à 20 minutes ;
c) on enlève l'agent de blanchiment de la surface de la dent ou des dents ;
d) et l'on reprend au moins une fois les étapes (a) à (c),
et lequel procédé comporte d'une à trois séances, étant entendu que dans chaque séance, l'on effectue les étapes (a) à (d), que l'on reprend les étapes (a) à (c) jusqu'à neuf fois par séance, et que l'on ajuste le nombre et la durée des reprises de manière à ce que la durée globale d'action de l'agent de blanchiment soit de 45 à 90 minutes par séance.

2. Procédé conforme à la revendication 1, dans lequel, dans l'étape (b), on laisse l'agent de blanchiment agir sur la dent ou les dents pendant 5 à 10 minutes.

3. Procédé conforme à la revendication 1 ou 2, dans lequel on reprend les étapes (a) à (c) jusqu'à cinq fois, et de préférence deux fois, par séance.

4. Procédé conforme à l'une des revendications 1 à 3, qui comporte au moins deux séances et dans lequel on laisse passer, entre deux séances consécutives, un laps de temps de 4 à 10 jours, et de préférence, de 6 à 8 jours.

5. Procédé conforme à l'une des revendications précédentes, dans lequel on ajuste le nombre et la durée des reprises de manière à ce que la durée globale d'action de l'agent de blanchiment soit de 60 minutes par séance.

6. Procédé conforme à l'une des revendications précédentes, dans lequel les dents à blanchir sont exemptes de dépôt.

7. Procédé conforme à l'une des revendications précédentes, dans lequel on recouvre d'un revêtement les zones qui ne doivent pas entrer en contact avec l'agent de blanchiment, en particulier la gencive, le bord de gencive et/ou les dents sensibles.

8. Procédé conforme à la revendication 7, dans lequel on utilise comme revêtement de la vaseline et/ou une digue dentaire

9. Procédé conforme à l'une des revendications précédentes, dans lequel on utilise un écarteur labial et jugal.

10. Procédé conforme à l'une des revendications précédentes, dans lequel on sèche la surface de la dent ou des dents avant d'y déposer l'agent de blanchiment.

11. Procédé conforme à l'une des revendications précédentes, dans lequel la composition contient, en tant que solvant, un alcool, en particulier de l'éthanol et/ou de l'isopropanol, de l'éther diéthylique, un ester, en particulier de l'acétate d'éthyle, et/ou de l'acétone, ainsi qu'un composé de type peroxyde en tant qu'agent oxydant pharmacologiquement admissible, et une alkyl-cellulose en tant qu'agent formant un vernis.

12. Procédé conforme à l'une des revendications 1 à 11, dans lequel la composition présente une viscosité de 5 à 20 000 mPa.s.

13. Procédé conforme à la revendication 11, dans lequel la composition contient une alkyl-cellulose dont la solubilité dans l'eau vaut au plus 0, 1 g dans 100 g d'eau à température ambiante.

14. Procédé conforme à l'une des revendications 1 à 13, dans lequel la composition contient en outre du dexpanthénol, de préférence en une proportion de 0,1 à 1,0 %, en poids rapporté au poids total de la composition.
